# EUROPEAN PATENT APPLICATION

(11) **EP 3 578 107 A1**
(43) Date of publication of application: **11.12.2019**
(21) Application number: 18176649.4
(22) Date of filing: 07.06.2018
(51) Int. Cl.: A61B 7/00, A61B 7/02, G01N 33/68

(54) **AN APPARATUS AND ASSOCIATED METHOD FOR USE IN DIAGNOSING A DIGESTIVE DISORDER**

(71) Applicant: Nokia Technologies Oy, 02610 Espoo (FI)
(72) Inventor: RADIVOJEVIC,, Zoran, Cambridge, CB3 0FA (GB)
(74) Representative: Potter Clarkson

(57) **Abstract**

An apparatus comprising means configured to:
determine, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
provide the determination of the presence and/or concentration of the analyte for use in diagnosing the digestive disorder of the human or animal body.

## Description

### Technical Field

The present disclosure relates to an apparatus and associated method for use in diagnosing a digestive disorder of a human or animal body. Some embodiments concern determining, based on a sound generated by reaction of a substance or composition within the digestive system of the human or animal body, one or more of the presence and concentration of an analyte indicative of a digestive disorder, and providing the determination of the presence and/or concentration of the analyte for use in diagnosing the digestive disorder.

Some embodiments may relate to portable electronic devices, in particular, so-called hand-portable electronic devices which may be hand-held in use (although they may be placed in a cradle in use). Such hand-portable electronic devices include so-called Personal Digital Assistants (PDAs) and tablet PCs. The portable electronic devices/apparatus according to one or more disclosed example aspects/embodiments may provide one or more audio/text/video communication functions (e.g. tele-communication, video-communication, and/or text transmission, Short Message Service (SMS)/ Multimedia Message Service (MMS)/emailing functions, interactive/non-interactive viewing functions (e.g. web-browsing, navigation, TV/program viewing functions), music recording/playing functions (e.g. MP3 or other format and/or (FM/AM) radio broadcast recording/playing), downloading/sending of data functions, image capture function (e.g. using a (e.g. in-built) digital camera), and gaming functions.

### Background

Research is currently being done to develop improved diagnostic techniques and equipment.

The listing or discussion of a prior-published document or any background in this specification should not necessarily be taken as an acknowledgement that the document or background is part of the state of the art or is common general knowledge.

### Summary

According to a first aspect, there is provided an apparatus comprising means configured to:
determine, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
provide the determination of the presence and/or concentration of the analyte for use in diagnosing the digestive disorder of the human or animal body.

The substance or composition may be configured to react with the analyte, and the means may be configured to determine the presence of the analyte based on detection of the generated sound.

The means may be configured to determine the concentration of the analyte based on one or more characteristics of the generated sound.

The means may be configured to determine the concentration of the analyte based on one or more of the amplitude and frequency of the generated sound.

The substance or composition may be configured to react on exposure to at least a minimum concentration of the analyte, and the means may be configured to determine that the analyte is present to at least the minimum concentration based on detection of the generated sound.

The ingestible body may further comprise a coating on the substance or composition which is configured to erode within a digestive time of the human or animal body on exposure of the coating to the analyte to reveal the underlying substance or composition for reaction with the analyte or another species within the digestive system, and the means may be configured to determine the presence of the analyte based on detection of the generated sound.

The coating may be configured to erode within a digestive time of the human or animal body on exposure of the coating to at least a minimum concentration of the analyte, and the means are configured to determine that the analyte is present to at least the minimum concentration based on detection of the generated sound.

The means may be configured to determine one or more of the presence and concentration of the analyte based on comparison of the generated sound with pre-calibrated audio data associated with one or more known analytes indicative of a digestive disorder.

The apparatus may comprise means configured to detect the generated sound.

The apparatus may comprise an interface configured to couple the apparatus to the skin of the human or animal body, the interface forming an acoustic cavity resonator configured to facilitate detection of the generated sound.

The interface may be a detachable or integral component of the apparatus.

The apparatus may be one or more of an electronic device, a portable electronic device, a portable telecommunications device, a mobile phone, a personal digital assistant, a tablet, a phablet, a desktop computer, a laptop computer, a server, a smartphone, a smartwatch, smart eyewear, smart earwear, and a module for one or more of the same.

According to a further aspect, there is provided a substance or composition for use in diagnosing a digestive disorder in a human or animal body, the substance or composition forming at least part of an ingestible body and configured to react on exposure of the ingestible body to an analyte indicative of the digestive disorder, reaction of the substance or composition within the digestive system of the human or animal body generating a detectable sound which enables one or more of the presence and concentration of the analyte to be determined.

The substance or composition may comprise one or more cavities which are opened on exposure of the ingestible body to the analyte to generate the detectable sound.

The ingestible body may further comprise a coating on the substance or composition which is configured to erode within a digestive time of the human or animal body on exposure of the coating to the analyte to reveal the underlying substance or composition for reaction with the analyte or another species within the digestive system.

One or more of the material and thickness of the coating may be configured to enable the coating to erode within a digestive time of the human or animal body on exposure of the coating to at least a minimum concentration of the analyte.

The coating may be configured to erode without generating a detectable sound.

The coating may be formed from the same material as, or a different material to, the substance or composition.

The coating may be formed by evaporation of a material onto the surface of the substance or composition.

The substance or composition may be formed as a sintered powder.

The substance or composition and the coating may be formed as respective sintered powders, and the sintered powder of the substance or composition may have a smaller particle size and be formed at a lower sintering pressure than the sintered powder of the coating.

The substance or composition may or may not comprise sodium bicarbonate.

The substance or composition may or may not comprise a pharmaceutically-active agent.

The substance or composition may comprise one or more of a chemical element, molecule, chemical compound, ion, alloy, mixture, solution, suspension and colloid.

The substance or composition may be one or more of a solid, liquid and gas.

The ingestible body may be one or more of a tablet, capsule and caplet.

The analyte may or may not comprise one or more of gastric acid, hydrochloric acid, hydrogen ions, hydroxide ions, bacteria, a virus, an enzyme, hydrogen sulphide, nitrogen, hydrogen, methane, and ammonia.

The other species in the digestive system with which the substance or composition may react (when the ingestible body comprises a coating configured to erode in the presence of the analyte) may comprise one or more of gastric acid, hydrochloric acid, hydrogen ions, hydroxide ions and an enzyme.

The digestive system may comprise one or more of the stomach, small intestine and large intestine.

According to a further aspect, there is provided use of a substance or composition in the manufacture of an ingestible body for the diagnosis of a digestive disorder in a human or animal body, the substance or composition forming at least part of the ingestible body and configured to react on exposure of the ingestible body to an analyte indicative of the digestive disorder, reaction of the substance or composition within the digestive system of the human or animal body generating a detectable sound which enables one or more of the presence and concentration of the analyte to be determined.

According to a further aspect, there is provided an apparatus comprising:
at least one processor; and
at least one memory including computer program code, the at least one memory and computer program code configured to, with the at least one processor, cause the apparatus at least to:
   determine, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
   provide the determination of the presence and/or concentration of the analyte for use in diagnosing the digestive disorder of the human or animal body.

According to a further aspect, there is provided a computer-implemented method comprising:
determining, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
providing the determination of the presence and/or concentration of the analyte for use in diagnosing the digestive disorder of the human or animal body.

According to a further aspect, there is provided a computer program comprising computer code configured to:
determine, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
provide the determination of the presence and/or concentration of the analyte for use in diagnosing the digestive disorder of the human or animal body.

According to a further aspect, there is provided an apparatus comprising means configured to:
determine, based on a sound generated by reaction of a substance or composition within the digestive system of a human or animal body, the concentration of an analyte within the digestive system, wherein the substance or composition forms at least part of an ingestible body and is configured to react on exposure of the ingestible body to the analyte; and
provide the determined analyte concentration for use in analysing one or more of the health and performance of the digestive system of the human or animal body.

According to a further aspect, there is provided a substance or composition for use in analysing one or more of the health and performance of the digestive system of a human or animal body, the substance or composition forming at least part of an ingestible body and configured to react on exposure of the ingestible body to an analyte within the digestive system, reaction of the substance or composition within the digestive system of the human or animal body generating a detectable sound which enables the concentration of the analyte to be determined.

According to a further aspect, there is provided use of a substance or composition in the manufacture of an ingestible body for analysing one or more of the health and performance of the digestive system of a human or animal body, the substance or composition forming at least part of the ingestible body and configured to react on exposure of the ingestible body to an analyte within the digestive system, reaction of the substance or composition within the digestive system of the human or animal body generating a detectable sound which enables the concentration of the analyte to be determined.

According to a further aspect, there is provided an apparatus comprising:
at least one processor; and
at least one memory including computer program code, the at least one memory and computer program code configured to, with the at least one processor, cause the apparatus at least to:
   determine, based on a sound generated by reaction of a substance or composition within the digestive system of a human or animal body, the concentration of an analyte within the digestive system, wherein the substance or composition forms at least part of an ingestible body and is configured to react on exposure of the ingestible body to the analyte; and
   provide the determined analyte concentration for use in analysing one or more of the health and performance of the digestive system of the human or animal body.

According to a further aspect, there is provided a computer-implemented method comprising:
determining, based on a sound generated by reaction of a substance or composition within the digestive system of a human or animal body, the concentration of an analyte within the digestive system, wherein the substance or composition forms at least part of an ingestible body and is configured to react on exposure of the ingestible body to the analyte; and
providing the determined analyte concentration for use in analysing one or more of the health and performance of the digestive system of the human or animal body.

According to a further aspect, there is provided a computer program comprising computer code configured to:
determine, based on a sound generated by reaction of a substance or composition within the digestive system of a human or animal body, the concentration of an analyte within the digestive system, wherein the substance or composition forms at least part of an ingestible body and is configured to react on exposure of the ingestible body to the analyte; and
provide the determined analyte concentration for use in analysing one or more of the health and performance of the digestive system of the human or animal body.

According to a further aspect, there is provided an ingestible body comprising any substance or composition described herein.

According to a further aspect, there is provided a system comprising any apparatus and ingestible body described herein.

The features described in relation to the first aspect are also applicable to the further aspects.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described example embodiments.

One or more of the computer programs may, when run on a computer, cause the computer to configure any apparatus, including a battery, circuit, controller, or device disclosed herein or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, or may be embodied as a transient signal. Such a transient signal may be a network download, including an internet download.

The present disclosure includes one or more corresponding aspects, example embodiments or features in isolation or in various combinations whether or not specifically stated (including claimed) in that combination or in isolation. Corresponding means for performing one or more of the discussed functions are also within the present disclosure.

The above summary is intended to be merely exemplary and non-limiting.

### Brief Description of the Figures

A description is now given, by way of example only, with reference to the accompanying drawings, in which:-
Figure 1 shows one example of the present apparatus;
Figure 2 shows a system comprising the present apparatus;
Figure 3a shows another example of the present apparatus comprising a detachable interface configured to couple the apparatus to the skin of a human or animal body;
Figure 3b shows another example of the present apparatus comprising an integral interface configured to couple the apparatus to the skin of a human or animal body;
Figure 4a shows one example of an ingestible body for use with the present apparatus;
Figure 4b shows another example of an ingestible body for use with the present apparatus;
Figure 5 shows a flowchart illustrating a method of using the present apparatus; and
Figure 6 shows a computer-readable medium comprising a computer program configured to perform, control or enable a method described herein.

### Description of Specific Aspects/Embodiments

Diagnosing disorders of the digestive system can be challenging. Taking images or a biopsy from the digestive tract is time-consuming, expensive and presents a health risk to the patient. So-called *"electronic pills"* are currently being developed for investigations of the digestive system. Electronic pills comprise miniature electronic modules, communication circuits, sensors and power sources, and are ingested by the patient to produce data which can be indicative of a digestive disorder. Some of these electronic pills, however, comprise materials which can be harmful to the patient if retained by the body.

There will now be described an apparatus and associated methods that may address one or more of the above-mentioned issues.

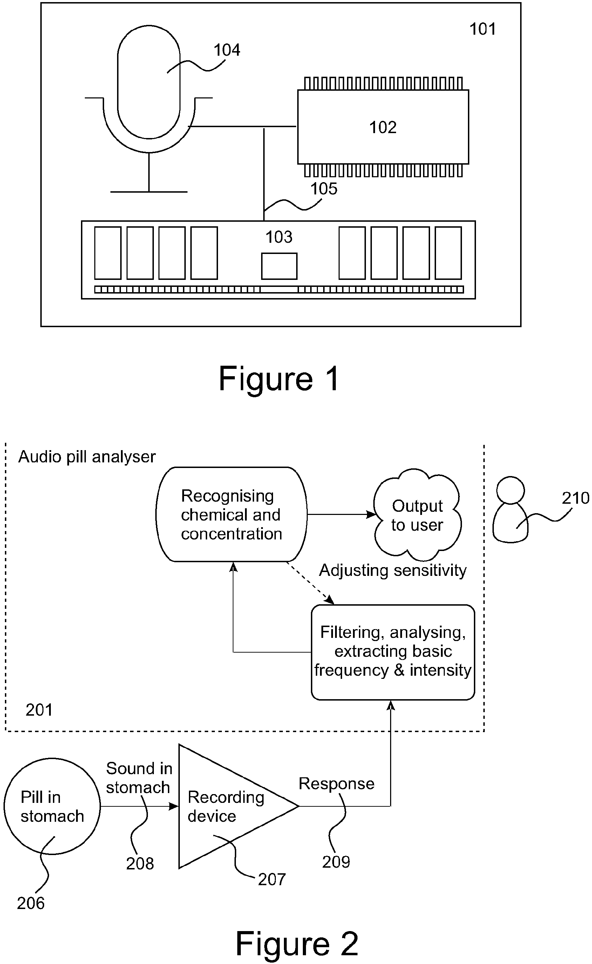

Figure 1 shows one example of the present apparatus 101. The apparatus 101 may be one or more of an electronic device, a portable electronic device, a portable telecommunications device, a mobile phone, a personal digital assistant, a tablet, a phablet, a desktop computer, a laptop computer, a server, a smartphone, a smartwatch, smart eyewear, smart earwear, and a module for one or more of the same.

The apparatus 101 comprises means configured to: determine, based on a sound generated by reaction of a substance or composition within the digestive system (e.g. stomach, small intestine and/or large intestine) of a human or animal body, one or more of the presence and concentration of an analyte indicative of a digestive disorder, wherein the substance or composition forms at least part of an ingestible body and is configured to react on exposure of the ingestible body to the analyte.

In the example of Figure 1, the means comprise a processor 102 and memory 103 which are connected to one another via a data bus 105. The memory 103 includes computer program code which is configured to, with the processor 102, cause the apparatus 101 to perform the above-mentioned functionality. The processor 102 may be configured for general operation of the apparatus 101 by providing signalling to, and receiving signalling from, the other components to manage their operation. The memory 103 may be configured to store computer code configured to perform, control or enable operation of the apparatus 101. The memory 103 may also be configured to store settings for the other components. The processor 102 may access the memory 103 to retrieve the component settings in order to manage the operation of the other components.

The means may be further configured to provide (e.g. make available) the determination of the presence and/or concentration of the analyte for use in diagnosing the digestive disorder of the human or animal body. In some example, the means may further comprise a transceiver (or a separate transmitter and receiver) configured to provide the determination of the presence and/or concentration of the analyte by transmission of the data to one or more other devices via a wired and/or wireless connection.

In the example of Figure 1, the apparatus 101 also comprises means 104 configured to detect the sound (such as fizzing, bubbling, outgassing or micro-explosions) generated by reaction of the substance or composition within the digestive system of the human or animal body. The means 104 are configured to detect the generated sound from outside the human or animal body (i.e. through the tissue), and may comprise one or more microphones and associated circuitry. For example, the means 104 may comprise a microphone configured to be positioned in proximity to the abdomen to enable the detection of sound generated from within one or more of the stomach, large intestine and small intestine.

Unlike the electronic pills mentioned above, the only technical requirement of the ingestible body (which may be referred to as an "audio pill") is that it comprises a substance or composition which can react within the digestive system on exposure of the ingestible body to the analyte to generate a detectable sound. As such, the ingestible body need not comprise any electronic components and may therefore be less expensive and pose less of a health risk than electronic pills.

Figure 2 shows a system comprising the present apparatus 201, the ingestible body 206 and a recording device 207. Unlike the apparatus 101 of Figure 1, the apparatus 201 in Figure 2 does not comprise means for detecting sound 208 generated by reaction of the substance or composition within the digestive system of the human or animal body. Instead, the recording device 207 is configured to record the generated sound 208 and transmit the recording 209 to the apparatus 201 for analysis. In this example, the apparatus 201 may be a server and the recording device 207 may be a smartphone.

In some cases, detection of the generated sound 208 may be sufficient to determine the presence of the target analyte in the digestive system of the human or animal body. For example, the substance or composition of the ingestible body 206 may be configured to react specifically with the target analyte resulting in the generation of a detectable sound 208. In this scenario, detection of the generated sound 208 serves as confirmation that the target analyte is present. In other cases, however, the substance or composition may be configured to react with several different analytes. As such, detection of the generated sound 208 only serves as an indication that one or more of these different analytes are present unless the generated sound is unique for each analyte. Further tests may therefore need to be performed to confirm the presence of the target analyte in this situation.

In order to determine the concentration of the target analyte, one or more characteristics of the generated sound 208 may need to be filtered, extracted and analysed by the present apparatus 201 (with possible adjustments for sensitivity). With some reactions, for example, the amplitude, intensity and frequency of the generated sound 208 are proportional to the concentration of the reactants. In this scenario, higher analyte concentrations may cause the substance or composition of the ingestible body 206 to dissolve more quickly and produce a louder sound at a higher frequency in comparison to lower analyte concentrations. One example of this is the reaction of sodium bicarbonate with hydrochloric acid which produces carbon dioxide, water and sodium chloride. If the substance or composition comprises sodium chloride, reaction of the sodium chloride with hydrochloric acid (target analyte) in the digestive system of the human or animal body will generate a bubbling sound as the carbon dioxide is produced. The amplitude and frequency of the sound generated in this example increases with the concentration of hydrogen ions (which is inversely proportional to the pH of the hydrochloric acid). As such, a measurement of the amplitude or frequency of the generated sound 208 can be used to determine the concentration of hydrogen ions, and thus, provide an indication of the pH of the hydrochloric acid. Similarly, the amplitude or frequency of the generated sound 208 may be used to determine the concentration of hydroxide ions in the digestive system as an indicator of pH.

In some cases, it may be possible to determine a minimum concentration of the analyte based on detection of the generated sound 208 rather than particular characteristics of the generated sound 208. For example, the substance or composition of the ingestible body 206 may be configured to react on exposure to at least a minimum concentration of the analyte. In this scenario, detection of the generated sound 208 would indicate that the analyte is present in the digestive system of the human or animal body to at least the minimum concentration. If the substance or composition was configured to react with gastric acid in the human body, detection of the generated sound 208 could be used to determine that the gastric acid contains at least the minimum concentration of hydrogen ions and therefore a pH below a corresponding threshold (which may be indicative of heartburn or indigestion).

In practice, the present apparatus 201 may be configured to determine the presence and/or concentration of the analyte based on comparison of the generated sound 208 with pre-recorded (and possibly pre-calibrated) audio data associated with one or more known analytes indicative of a digestive disorder. This may be particularly useful when the substance or composition of the ingestible body 206 is able to react with several different analytes that could potentially be present in the digestive system of the human or animal body at the same time. If each reaction generates a different detectable sound 208, then comparison of the generated sound 208 with the pre-recorded audio data can help to identify the particular analyte present. Furthermore, comparison of the generated sound 208 with pre-calibrated audio data can enable a more quantitative measurement of the concentration (or pH for aqueous solutions) of the target analyte.

Once the presence and/or concentration of the target analyte have been determined, the apparatus 201 may be configured to output this determination to a user 210. The user 210 may be a human patient under investigation or a medical practitioner (e.g. doctor, nurse, vet) investigating a human or animal patient.

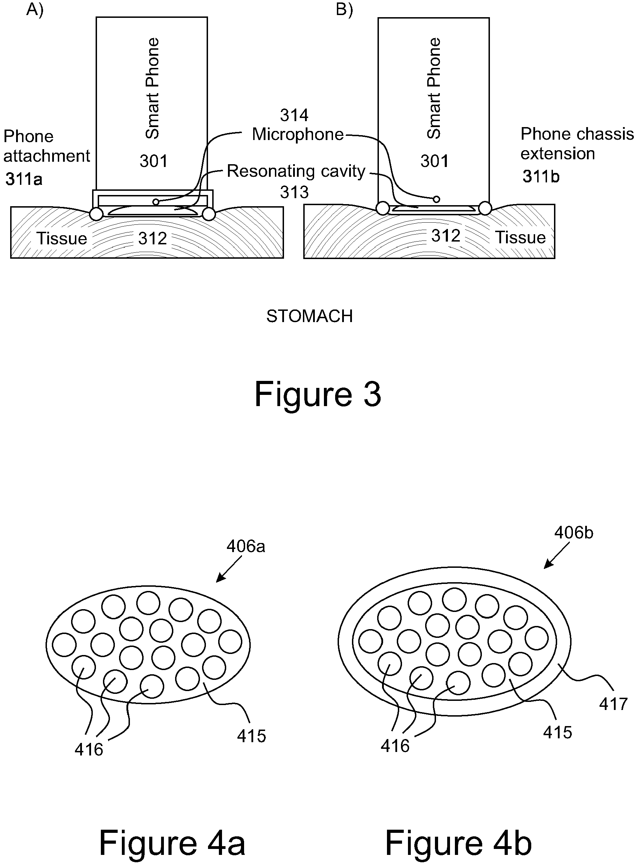

Figures 3a and 3b show further examples of the present apparatus 301. In these examples, the apparatus 301 comprises an interface 311a,b configured to couple the apparatus 301 to the skin 312 of a human or animal body. The interface 311a,b may be detachable from the apparatus 301 (Figure 3a) or integral to the apparatus 301 (Figure 3b) and forms an acoustic cavity resonator 313 configured to facilitate detection of the generated sound. In either case, the interface 311a,b may be formed from a resilient material (e.g. elastomeric silicone) which can conform to the skin 312 of the human or animal body to define the cavity 313 between the apparatus 301 and the skin 313. The apparatus 301 shown in Figures 3a and 3b is a smartphone comprising an in-built microphone 314 for detecting the generated sound, and the interface 311 a,b is configured to form a cavity 313 which encloses the entrance to the microphone 314. This configuration helps to reduce the amount of background noise detected by the apparatus 301 (i.e. passive noise cancellation). In some cases, the apparatus 301 may also comprise active noise cancellation to further reduce the detection of background noise. A holster (not shown) may be used to hold the apparatus 301 in position during detection of the generated sound to reduce background noise caused by relative movement between the apparatus 301 and skin 312. This may be particularly beneficial for recording digestive sounds over prolonged periods of time.

Figure 4a shows one example of an ingestible body 406a for use with the present apparatus. The ingestible body 406a may be one or more of a tablet, capsule and caplet for oral administration by a human or animal patient. In this example, the ingestible body 406a comprises a substance or composition 415 configured to react with a target analyte within the digestive system of the human or animal body to generate a detectable sound. The substance or composition 415 may be configured to react at any concentration of the analyte, or it may only react when the analyte concentration exceeds a minimum concentration.

The substance or composition 415 may comprise one or more of a chemical element, molecule, chemical compound, ion and alloy depending on the particular analyte of interest. In some cases, the substance or composition 415 may comprise a pharmaceutically-active agent for treating a digestive disorder of the human or animal body. In this scenario, detection of the generated sound confirms successful delivery of the pharmaceutically-active agent to the patient's digestive system.

As shown in Figure 4a, the substance or composition 415 of the ingestible body in this example also comprises one or more cavities 416 which are opened on exposure of the ingestible body 406a to the analyte to generate the detectable sound. As the reaction between the substance or composition 415 and the analyte progresses, the analyte gradually dissolves the substance or composition 415 and penetrates the cavities 416. When the cavities 416 are opened, the surface area exposed to the analyte increases causing acceleration of the reaction and an increase in one or more of the amplitude, intensity and frequency of the associated sound.

Figure 4b shows another example of an ingestible body 406b for use with the present apparatus. In this example, the ingestible body 406b further comprises a coating 417 on the substance or composition 415 which is configured to erode within a digestive time of the human or animal body on exposure of the coating 417 to the analyte to reveal the underlying substance or composition 415. The substance or composition 415 may be configured to generate a detectable sound on reaction with the analyte or another species in the digestive system. This may be useful, for instance, when erosion of the coating 417 in the presence of the analyte does not generate a detectable sound itself. For example, the coating 417 may be configured to erode silently in the presence of a particular pathogen, and the underlying substance or composition 415 may comprise sodium bicarbonate suitable for reacting with hydrochloric acid in the stomach. Detection of the characteristic bubbling sound produced by the generation of carbon dioxide may therefore be used to indicate that the pathogen is present in the digestive system of the human or animal body. In some cases, one or more of the material and thickness of the coating 417 may be configured to enable the coating 417 to erode within a digestive time of the human or animal body on exposure of the coating 417 to at least a minimum concentration of the analyte. In this scenario, detection of the sound generated by reaction of the underlying substance or composition 415 may be used to indicate that the analyte is present to at least the minimum concentration.

The coating 417 may be formed from a different material to the substance or composition 415. On the other hand, the coating 417 may be formed from the same material but with a different physical structure. For example, the substance or composition 415 and the coating 417 may be formed as respective sintered powders of the same material such that the sintered powder of the substance or composition 415 has a smaller particle size and is formed at a lower sintering pressure than the sintered powder of the coating 417. The rate of reaction of the material increases as the particle size and sintering pressure decrease. As such, the coating 417 may be used to delay reaction of the substance or composition 415 until the ingestible body 406b has reached a particular point (e.g. small or large intestine rather than stomach) in the digestive system. Furthermore, the lower reaction rate associated with the coating 417 may inhibit the generation of a detectable sound until exposure of the underlying substance or composition 415.

The substance or composition 415 and coating 417 may be formed using a variety of techniques other than sintering. For example, the substance or composition 415 may be formed using one or more of powder blending, milling, granulation and hot melt extrusion, and the coating 417 may be formed by evaporation of a material onto the surface of the (pre-formed) substance or composition 415. In addition, the substance or composition 415 may comprise a fluid (liquid and/or gas), and the coating 417 may form a hard-shelled or soft-shelled capsule configured to contain the fluid. The fluid may comprise one or more of a mixture, solution, suspension and colloid.

The apparatus and ingestible body described herein may be configured to detect a variety of different analytes indicative of a digestive disorder of a human or animal body. As mentioned previously, the ingestible body could be used to determine the concentration of hydrogen or hydroxide ions in the stomach, and thus, the pH of the gastric fluid. In this example, the patient could ingest an antacid to neutralise the gastric fluid before ingesting the ingestible body. A sound recording in this pH-neutral state could then be used to provide a reference audio signal for comparison with subsequently detected sounds. This example may be useful in the diagnosis of one or more of heartburn and indigestion.

Other applications of the present apparatus and ingestible body are in the diagnosis of gut flora imbalance, irritable bowel syndrome and food intolerance. These conditions typically cause the production of excessive amounts of hydrogen sulphide, nitrogen, hydrogen, methane and/or ammonia. If one or more of the substance or composition and coating are configured to react with at least one of these chemicals resulting in the generation of a detectable sound, then the detected sound may be used to determine the presence and/or concentration of these chemicals. In practice, a sound recording taken before a meal could be used as a reference audio signal for comparison with sounds detected after a meal to help identify any digestive issues triggered by that particular meal.

Furthermore, the apparatus and ingestible body may be configured to detect predetermined bacteria or viruses known to cause digestive disorders in the human or animal body, or they may be configured to detect a particular enzyme required for a healthy digestive system. In the case of bacteria or viruses, determination of the presence of the analyte may be sufficient to identify a digestive disorder. In the case of enzymes, on the other hand, determination of an enzyme concentration below a predefined threshold associated with a healthy digestive system may be required to identify a digestive disorder.

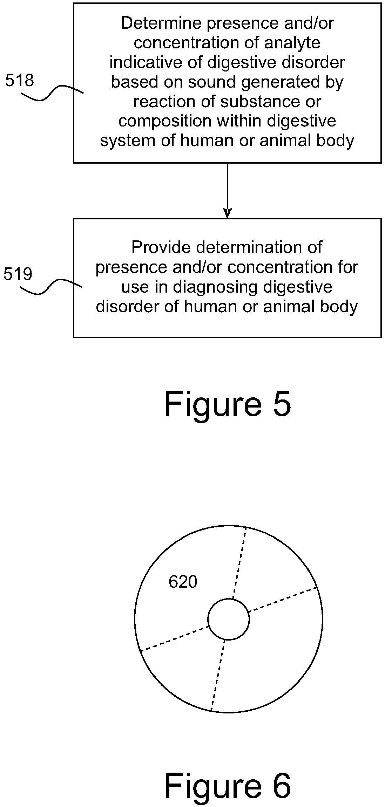

Figure 5 shows the main steps 518-519 of a computer-implemented method of using the present apparatus. The method generally comprises: determining, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte 518; and providing the determination of the presence and/or concentration of the analyte for use in diagnosing the digestive disorder of the human or animal body 519.

Figure 6 illustrates schematically a computer/processor readable medium 620 providing a computer program according to one embodiment. The computer program may comprise computer code configured to perform, control or enable one or more of the method steps 518-519 of Figure 5 using at least part of the apparatus described herein. In this example, the computer/processor readable medium 620 is a disc such as a digital versatile disc (DVD) or a compact disc (CD). In other embodiments, the computer/processor readable medium 620 may be any medium that has been programmed in such a way as to carry out an inventive function. The computer/processor readable medium 620 may be a removable memory device such as a memory stick or memory card (SD, mini SD, micro SD or nano SD).

Although particular emphasis has been placed on the detection of analytes indicative of a digestive disorder, the apparatus and ingestible body described herein may also be used to determine the concentration of an analyte within the digestive system for use in analysing one or more of the health and performance of the digestive system of the human or animal body. This may be useful for users/patients who wish to monitor the digestion of their meals to identify one or more of the most suitable food types, food combinations, portions sizes and meal times for their digestive systems (regardless of the presence of any digestive disorders). Over time, this information may be used to develop a personalised diet.

Other embodiments depicted in the figures have been provided with reference numerals that correspond to similar features of earlier described embodiments. For example, feature number 1 can also correspond to numbers 101, 201, 301 etc. These numbered features may appear in the figures but may not have been directly referred to within the description of these particular embodiments. These have still been provided in the figures to aid understanding of the further embodiments, particularly in relation to the features of similar earlier described embodiments.

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some embodiments, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such embodiments can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some embodiments one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/embodiments may consist of any such individual feature or combination of features. In view of the foregoing description it will be evident to a person skilled in the art that various modifications may be made within the scope of the disclosure.

While there have been shown and described and pointed out fundamental novel features as applied to different embodiments thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment may be incorporated in any other disclosed or described or suggested form or embodiment as a general matter of design choice. Furthermore, in the claims means-plus-function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising means configured to:
determine, based on a sound generated by reaction of a substance or composition within the digestive system of a human or animal body, one or more of the presence and concentration of an analyte indicative of a digestive disorder, wherein the substance or composition forms at least part of an ingestible body and is configured to react on exposure of the ingestible body to the analyte; and
provide the determination of the presence and/or concentration of the analyte for use in diagnosing the digestive disorder of the human or animal body.

2. The apparatus of claim 1, wherein the substance or composition is configured to react with the analyte, and wherein the means are configured to determine the presence of the analyte based on detection of the generated sound.

3. The apparatus of claim 2, wherein the means are configured to determine the concentration of the analyte based on one or more characteristics of the generated sound.

4. The apparatus of claim 3, wherein the means are configured to determine the concentration of the analyte based on one or more of the amplitude and frequency of the generated sound.

5. The apparatus of any of claims 2 to 4, wherein the substance or composition is configured to react on exposure to at least a minimum concentration of the analyte, and wherein the means are configured to determine that the analyte is present to at least the minimum concentration based on detection of the generated sound.

6. The apparatus of claim 1, wherein the ingestible body further comprises a coating on the substance or composition which is configured to erode within a digestive time of the human or animal body on exposure of the coating to the analyte to reveal the underlying substance or composition for reaction with the analyte or another species within the digestive system, and wherein the means are configured to determine the presence of the analyte based on detection of the generated sound.

7. The apparatus of claim 6, wherein the coating is configured to erode within a digestive time of the human or animal body on exposure of the coating to at least a minimum concentration of the analyte, and wherein the means are configured to determine that the analyte is present to at least the minimum concentration based on detection of the generated sound.

8. The apparatus of any preceding claim, wherein the apparatus comprises means configured to detect the generated sound, and wherein the apparatus comprises an interface configured to couple the apparatus to the skin of the human or animal body, the interface forming an acoustic cavity resonator configured to facilitate detection of the generated sound.

9. A substance or composition for use in diagnosing a digestive disorder in a human or animal body, the substance or composition forming at least part of an ingestible body and configured to react on exposure of the ingestible body to an analyte indicative of the digestive disorder, reaction of the substance or composition within the digestive system of the human or animal body generating a detectable sound which enables one or more of the presence and concentration of the analyte to be determined.

10. An ingestible body comprising the substance or composition of claim 9.

11. The ingestible body of claim 10, wherein the substance or composition comprises one or more cavities which are opened on exposure of the ingestible body to the analyte to generate the detectable sound.

12. The ingestible body of claim 10 or 11, wherein the ingestible body further comprises a coating on the substance or composition which is configured to erode within a digestive time of the human or animal body on exposure of the coating to the analyte to reveal the underlying substance or composition for reaction with the analyte or another species within the digestive system.

13. The ingestible body of any of claims 10 to 12, wherein the coating is configured to erode without generating a detectable sound.

14. The ingestible body of any of claims 10 to 13, wherein the substance or composition is formed as a sintered powder.

15. The ingestible body of claim 14 when dependent upon claim 13, wherein the substance or composition and the coating are formed as respective sintered powders, and wherein the sintered powder of the substance or composition has a smaller particle size and is formed at a lower sintering pressure than the sintered powder of the coating.
